# EUROPEAN PATENT SPECIFICATION

(11) **EP 1 992 309 B1**
(45) Date of publication and mention of the grant of the patent: **20.04.2011**
(21) Application number: 08005749.0
(22) Date of filing: 27.03.2008
(51) Int. Cl.: A61F 2/38, A61F 2/00, A61F 2/30

(54) **Implant articular surface wear reduction system**
System zur Verschleißminderung von Gelenkoberflächenimplantaten
Système de réduction d'usure d'une surface articulaire à implant

(30) Priority: 16.05.2007 US 749598
(43) Date of publication of application: 19.11.2008
(73) Proprietor: Zimmer, Inc., Warsaw IN 46580 (US)
(72) Inventor: Scrafton, Joel, Syracuse Indiana 46567 (US); Popoola, Oludele, Granger Indiana 46530 (US)
(74) Representative: Mays, Julie

(56) References cited:
- EP-A- 0 519 872
- WO-A-03/049781
- WO-A-2007/109714
- DE-A1- 10 051 437
- DE-U1-202006 012 902
- US-A- 5 180 394
- US-A1- 2007 032 877
- US-B1- 6 203 844

## Description

### FIELD OF THE INVENTION

This invention relates to implant devices, and, more particularly, to an implant articular surface wear reduction system comprising a polymeric implant component, a rigid implant component, and a hard wear layer.

### BACKGROUND OF THE INVENTION

The long-term performance of a prosthetic implant depends on the resistance that the implant has to the extreme conditions found in the human body. Proper material selection is paramount in preventing premature implant failure. In addition to the chemical and biological imposed material challenges, articulating surfaces on orthopedic implants have an additional abrasion resistance requirement. Prosthetic implants must be designed to meet these challenges.

Prosthetic implants, such as those used in a total joint arthroplasty, generally have multiple articulating surfaces that slide and rotate in contact with one another, often while under a load. So, the articulating surface must first resist degradation from attack by the biological fluids, and second, the surfaces must resist wear due to continuous frictional contact with an opposing articulating surface. For example, in total knee arthroplasty, a prosthetic implant replaces a femoro-tibial joint, commonly called a knee. The knee is a complex joint between multiple bones and a group of ligaments. Two of the primary bones are a femur and a tibia. The knee exists at a junction between the ends of those two bones. Therefore, in knee replacement, the prosthetic implant replaces a portion of each of the femur and the tibia. The synthetic materials of the prosthesis specifically replace the condylar surfaces of the femur and condylar surfaces of a tibia. The prosthetic joint also replaces the two meniscuses (i.e. the medial and lateral meniscuses) situated between the condylar surfaces of the femur and the tibia. The meniscuses are made of fibrocartilage, which gives them elastic properties. The meniscuses function to improve the fit between the condyles of the femur and the tibia, to absorb shock and distribute load in the knee, and to help move lubricating fluid around the knee. The prosthetic implant must mimic the natural kinematics of the original knee component including the function of the meniscuses.

The kinematic motions of the knee are not simple. Knee flexion/extension involves a combination of rolling and sliding called femoral rollback. The condyles are specially adapted to perform this asymmetrical motion. Because of asymmetry between the lateral and medial femoral condyles, the lateral condyle rolls a greater distance than the medial condyle during knee flexion. The sliding and rolling motions, in conjunction with complex loading conditions, creates a unique and a demanding problem for any knee prosthesis.

Medical professionals and patients have similar goals when considering a total knee arthroplasty. Of course, the first goal is to eliminate the dysfunctional joint, whether due to arthritis, disease, or injury. Another goal is to achieve an optimum level of performance. Ideally, the prosthetic implant plant must provide nearly the same kinematic motion as the natural joint. At the same time the prosthesis should be durable because total joint arthroplasty is a serious surgical procedure. It is preferable that the joint should outlast its host. In addition to their resistance to biodegradation, the performance of a prosthetic implant is also assessed by its resistance to wear. The biological cellular reactions to wear particles have been found to cause bone resorption which results in implant loosening and, consequently, corrective surgeries. Traditional metal or polyethylene knee implants consist of metallic femoral components and polymeric monoblocks or tibial trays.

US-A-5 180 398 relates to an implant wear reduction system as set out in the pre-characterising portion of claim 1. However, this document does not disclose a mounting surface including a first recess and a hard wear layer positioned in the recess.

DE 100 51 437 A1 describes a tibial component of a knee prosthesis which is made of a graded polymer material. The polymer material has a hard surface and elastic structures located under the surface. US-B1-6 203 844 relates to a polymeric prosthesis pre-coated with a bone cement compatible polymer. WO 03/049781 A describes a surface for a prosthesis comprising a metallic material with a textured surface and oxidation layer coating. DE 20 2006 012902 U1 relates to an implant comprising an implant material, an artificial joint surface and a wear-reducing hard coating. EP-A-0 519 872 relates to a wear-reducing knee prosthesis. US 2007/032877 A1 describes a prosthetic joint having first and second bearing members, at least one of which has a ceramic substrate and a carbon coating. WO 2007/103714 A describes prosthetic joints comprising a metal, ceramic or polymer substrate and a protection coating of tetrahedral bonded carbon. None of these documents a disclose a mounting surface including a first recess and a hard wear layer positioned in the recess.

Therefore, what is needed in the art is an implant articular surface wear reduction system. The implant articular surface wear reduction system must reduce wear between implant articulation surfaces, and thus improve the longevity of the prosthetic implant. In addition, the implant articular surface wear reduction system must permit natural kinematic motion and provide a low-friction, durable prosthetic joint.

### SUMMARY OF THE INVENTION

The present invention provides an implant articular surface wear reduction system which improves orthopedic prosthetic joint longevity by reducing frictional abrasive wear. The system includes a rigid implant component and a polymeric implant component. The rigid implant component has a working surface. In one embodiment, the working surface is one opposing articular surface of a joint. The polymeric plant component has a mounting surface to which a hard wear layer is attached. The hard wear layer represents the other opposing articular surface of the joint. Thus, motion of the rigid implant component may cause the working surface of the rigid implant component to move while in contact with the opposing articular surface on the hard wear layer. The mounting surface on the polymeric implant component has a first recess such that the hard wear layer is positioned within the first recess. In one embodiment, the rigid implant component and the hard wear layer comprise ceramic material. In another embodiment, the rigid implant component comprises a metal, and a non-metallic wear layer is attached to the working surface. In this embodiment the non-metallic wear layer represents an opposing articular surface of a joint.

In one embodiment, the working surface on the rigid implant component has a second recess such that the non-metallic wear layer is positioned within the second recess.

In another embodiment, the polymeric implant component is a tibial implant and the hard wear layer is a metal or a ceramic, and the rigid implant component is a femoral implant and the non-metallic wear layer is a polymer, such as polyethylene, PEEK, PEKK, polypropylene, or PrimoSpire™ self-reinforced polyphenylene (SRP).

In yet another embodiment, the opposing articular surfaces retain fluid interposed between the two articular surfaces.

### BRIEF DESCRIPTION OF THE DRAWINGS

The accompanying drawings, which are incorporated in and constitute a part of this specification, illustrate embodiments of the invention and, together with a general description of the invention given above, and the detailed description given below, serve to explain the invention.

FIG. 1A is a perspective view of one embodiment of a rigid implant component;

FIG. 1B is a disassembled perspective view of one embodiment of a polymeric implant component and a hard wear layer positioned for attachment to the component;

FIG. 1C is an assembled perspective view of the embodiment of the invention depicting the rigid implant component from FIG. 1A attached to a femur and the polymeric implant component from FIG. 1B attached to a tibia.

FIG. 2 is a disassembled view of one embodiment of the rigid implant component and a non-metallic wear layer positioned for attachment to the component;

FIG. 2A is a partial perspective view of an encircled area 1A of FIG. 2 of one embodiment of a non-metallic wear layer including a ceramic coating;

FIG. 3 is an assembled perspective view of the embodiment of the invention depicting the rigid implant component from FIG. 2 attached to a femur and the polymeric implant component from FIG. 1B attached to a tibia.

### DETAILED DESCRIPTION

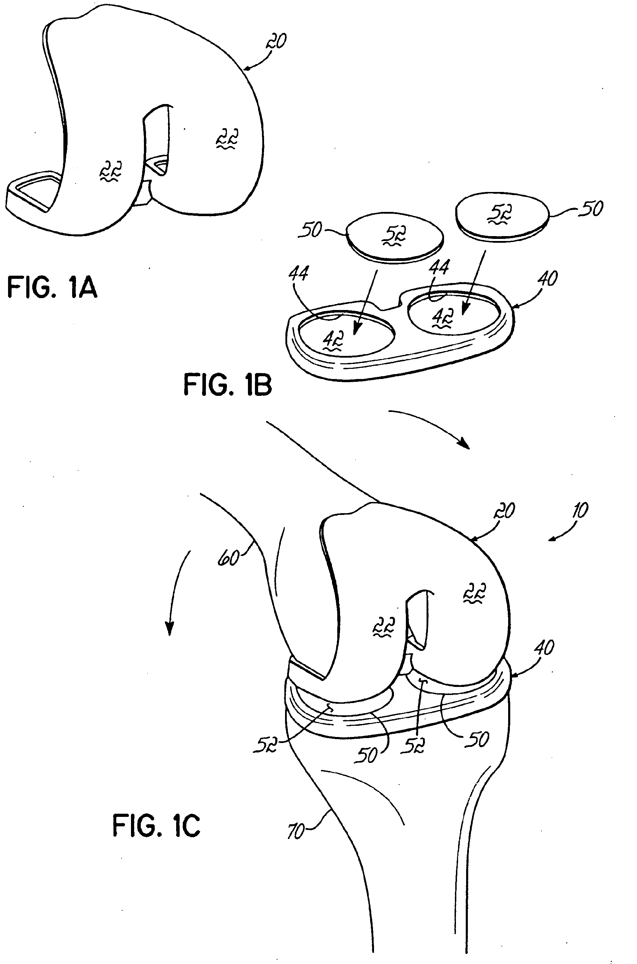

With reference generally to FIGS. 1A - 3, an implant articular surface wear reduction system 10 has a rigid implant component 20 and a polymeric implant component 40. FIG. 1A illustrates one embodiment of the rigid implant component 20. The rigid implant component 20 has a working surface 22. In one embodiment of the implant articular surface wear reduction system 10, the rigid implant component 20 comprises a ceramic. By way of example and not limitation, the ceramic may be an oxide ceramic, such as alumina or zirconia; non-oxide ceramic, such as silicon nitride or silicon carbide; or other ceramic materials that are biologically inert, and yet are sufficiently hard and abrasion resistant. In addition, the ceramic may be a monolith or, alternatively, the ceramic may be a plurality of discrete micro- or macroscopic particles held in a matrix. In another embodiment, the rigid implant component 20 comprises a metal with a ceramic coating (not shown) on the working surface 22. The ceramic coating may be sprayed onto the working surface 22 by, for example, plasma spray, flame spray, HVOF spray, cold spray, or other spray coating technique that provides bonding to the working surface 22.

FIG. 1B illustrates an embodiment of the polymeric implant component 40. The polymeric implant component 40 has a mounting surface 42. A hard wear layer 50 is attached to the mounting surface 42, and the hard wear layer 50 has a hard articular surface 52. As one skilled in the art will appreciate, the hard wear layer 50 may be attached to the polymeric implant component 40 with an appropriate adhesive, welding, or other biologically inert and resistant means. By way of example and not limitation, the polymeric implant component 40 may comprise polyethylene, including ultra high weight molecular weight polyethylene (UHMW PE); polyetheretherketone (PEEK); polyetherketoneketone (PEKK); polycarbonate urethane (PCU); or PrimoSpire^{™} self-reinforced polyphenylene (SRP), available from SOLVAY Advanced Polymers, LLC, Alpharetta, Georgia; or other biologically inert or compatible polymers.

In one embodiment of the polymeric implant component 40, as shown in FIG. 1B, the polymeric implant component 40 may have a first recess 44. The mounting surface 42 may reside within the first recess 44. The hard wear layer 50 may be secured within the first recess 44. Shear stresses on a hard wear layer-to-polymeric implant component interface may be borne, in part, by the first recess 44. Thus, the first recess 44 may substantially prevent the hard wear layer 50 from detaching from the polymeric implant component 40. By way of example, in some embodiments of the present invention, the polymeric implant component 40 may be formed around the hard wear layer 50 by injection molding, or the like, to create an intimate, load-bearing interface between the polymeric implant component 40 and the hard wear layer 50.

In one embodiment of the invention, the hard wear layer 50 attached to the polymeric implant component 40 is a ceramic insert. As shown in FIG. 1B, the ceramic insert may be attached within the first recess 44 in the polymeric implant component 40. By way of example and not limitation, the ceramic insert may be an oxide ceramic, such as alumina or zirconia; non-oxide ceramic, such as silicon nitride or silicon carbide; or other ceramic materials that are biologically inert, and yet are sufficiently hard and abrasion resistant. In addition, the ceramic insert may be a monolith or, alternatively, the ceramic insert may be a plurality of discrete micro- or macroscopic particles held in a matrix.

FIG. 1C illustrates the system 10 where the rigid implant component 20 is adapted to be attached to a first bone 60, and the polymeric implant component 40 is adapted to be attached to a second bone 70 where the first and second bones 60, 70 meet to form a natural articular joint. As shown in FIG. 1C, the rigid implant component 20 is adapted to rotate and slide against the hard wear layer 50. The polymeric implant component 40 may exhibit some elasticity during loading to allow axial deflection or deformation of the polymeric implant component 40 between the bone 70 and the hard wear layer 50. When the system 10 is loaded by activity, such as running, throwing, lifting, swimming, or pulling, the pressures exerted are transferred through the system 10 causing the working surface 22 to articulate against the hard wear layer 50. In particular, loads may pass from the first bone 60 to the rigid implant component 20, through the hard wear layer 50, through the polymeric implant component 40 and into the second bone 70. Pressures exerted on the bones 60, 70 may be partially absorbed and dispersed by the polymeric implant component 40 with little or no wear of the working surface 22 or the hard wear layer 50.

In another embodiment of the invention, a lubricating fluid (not shown) may be retained between the working surface 22 and the hard articular surface 52. Each of the working and hard articular surfaces 22, 52 individually or in combination may be treated, honed, dimpled, or otherwise provided with surface topography which retain the lubricating fluid between the working and hard articular surfaces 22, 52.

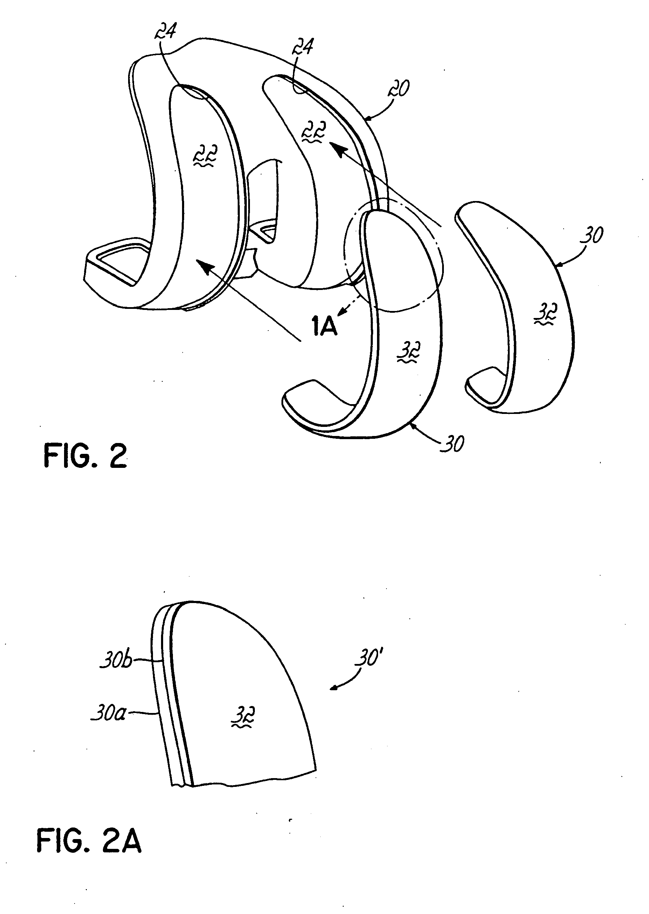

Another embodiment of the rigid implant component 20 is illustrated in FIG. 2. As shown, a non-metallic wear layer 30 is attached to the working surface 22. Furthermore, the non-metallic wear layer 30 has a non-metallic articular surface 32. As one skilled in the art will appreciate, the non-metallic wear layer 30 may be attached to the rigid implant component 20 with an appropriate adhesive, welding, or other biologically inert and resistant means. By way of example and not limitation, the rigid implant component 20 may comprise titanium, a titanium alloy, a cobalt chromium alloy, a zirconium alloy, a stainless steel, or other biocompatible metal.

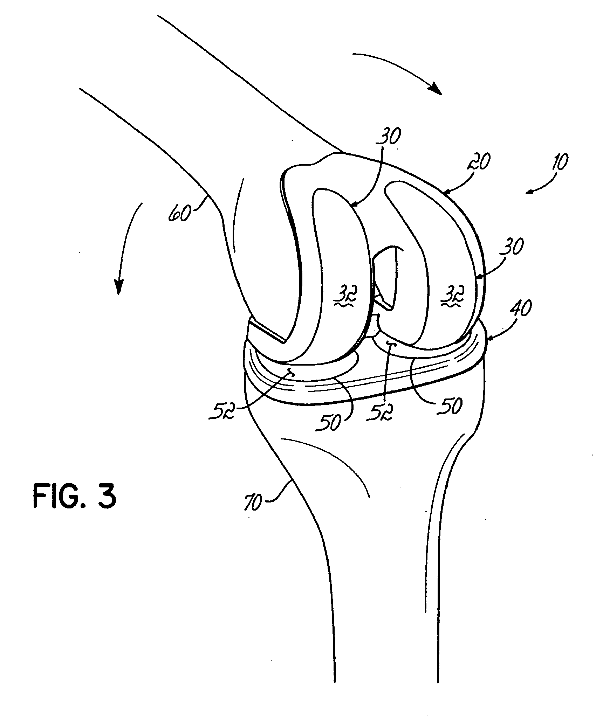

As shown in FIG. 3 the non-metallic wear layer 30, which is attached to the:rigid implant component 20, is adapted to rotate and slide against the hard wear layer 50. Similar to the embodiment shown in FIG. 1C, the polymeric implant component 40 may exhibit some elasticity during loading to allow axial deflection or deformation of the polymeric implant component 40 between the bone 70 and the hard wear layer 50. The non-metallic wear layer 30 may provide elasticity or flexibility between hard wear layer 50 and the rigid implant component 20. Therefore, pressures exerted on the bones 60, 70 may be partially absorbed during elastic motion of the polymeric implant component 40 and the non-metallic wear layer 30. In addition, deformation of the non-metallic wear layer 30 at the hard wear layer-to-non-metallic wear layer interface may distribute applied loads across a greater area, which will lessen the pressures at this interface.

In another embodiment of the invention, the hard wear layer 50 attached to the polymeric implant component 40 is a metal insert. Similar to the ceramic insert, as discussed above, the metal insert may be attached within the first recess 44 in the polymeric implant component 40. The metal insert is adapted to articulate against the non-metallic wear layer 30. By way of example and not limitation, the metal insert may comprise titanium, a titanium alloy, a cobalt chromium alloy, stainless steel, or other biocompatible metal having sufficient hardness and resistance to the *in vivo* environment. The metal insert may be configured as a single component or may be multiple metallic pieces arranged within the first recess 44 of the polymeric implant component 40. Furthermore, the hard wear layer 50 may be provided with coatings, such as TiN, Cr₂N, and Diamond-Like Coatings (DLC), as are known in the art, to further enhance their durability.

During a total joint arthroscopic procedure, the system 10 is implanted *in vivo* and replaces the natural articular joint. Specifically, and as shown in FIGS. 1C and 3, the rigid implant component 40 is attached to a portion of the first bone 60, and the polymeric implant component 20 is attached to a portion of the second bone 70. Any cartilage between the first and second bones 60, 70 is removed. Thus, once a surgeon installs the implant articular surface wear reduction system 10, the rigid implant component 20 and the polymeric implant component 40 are positioned substantially adjacent to one another. The system 10, therefore, replaces portions of the first and the second bone surfaces of the natural articular joint with the non-metallic articular surface 32 and hard articular surface 52.

The non-metallic articular surface 32 and the hard articular surface 52 permit the sliding and rotating motion similar to the natural articular joint. When the system 10 is loaded by activity, such as running, throwing, lifting, swimming, or pulling, the pressures exerted are transferred through the system 10 causing the non-metallic wear layer 30 to articulate against the hard wear layer 50. In particular, loads may pass from the first bone 60 to the rigid implant component 20 through the non-metallic wear layer 30, through the hard wear layer 50, through the polymeric implant component 40 and into the second bone 70. Pressures exerted on the bones 60, 70 may be partially absorbed and dispersed by the polymeric implant component 40 and the non-metallic wear layer 30 due to their elasticity. Overall, the system 10 may act to distribute the pressures across a larger area and lessen the pressures at the hard wear layer-to-non-metallic wear layer interface. The non-metallic wear layer 30 and the hard wear layer 50 slide and rotate against one another and yet substantially little wear is observed. Moreover, in another embodiment of the invention, the lubricating fluid (not shown) may be retained between the non-metallic articular surface 32 and the hard articular surface 52. Each of the non-metallic and hard articular surfaces 32, 52 individually or in combination may be treated, honed, dimpled, or otherwise provided with surface topography which retain the lubricating fluid between the non-metallic and hard articular surfaces 32, 52.

In one embodiment of the invention, the rigid implant component 20 may be characterized as occupying a volume that is substantially greater than a volume of the non-metallic wear layer 30. Thus, the rigid implant component 20 may not substantially deflect while under load. Rigidity of the rigid implant component 20 may permit uniform, predictable, and consistent loading of the non-metallic wear layer 30. Consequently, when loaded, the non-metallic articular surface 32 receives substantially uniform frictional contact with the hard wear layer 50, and thus, rigidity of the rigid implant component 20 may limit abrasion along any specific portion of the non-metallic articular surface 32.

With reference once again to FIG. 2, in one embodiment, the rigid implant component 20 has a second recess 24. The second recess 24 is formed such that the working surface 22 is positioned within the second recess 24. The second recess 24 may cooperate with the non-metallic wear layer 30 such that the non-metallic wear layer 30 may be inserted therein. The second recess 24 may then structurally support the non-metallic wear layer 30 during use. Furthermore, shear stresses on the non-metallic wear layer-to-rigid implant component interface may be distributed to the second recess 24, and at least partially to the rigid implant component 20. In addition, detachment of the non-metallic wear layer 30 may be avoided by more fully supporting the non-metallic wear layer 30 with the second recess 24. As one skilled in the art will observe and appreciate, the non-metallic wear layer 30 may be formed within the second recess 24, such as, by injection molding or other forming technique such that the non-metallic wear layer 30 substantially fills the second recess 24 and is bonded to the rigid implant component 20.

In another embodiment of the invention, the non-metallic wear layer 30 attached to the rigid implant component 20 may be a polymeric insert. As shown in FIG. 2A, the polymeric insert may be attached within the second recess 24 in the rigid implant component 20. By way of example and not limitation, the polymer of the polymeric insert may comprise polyethylene (PE) or any one of a number of other biocompatible polymeric materials that generally exhibit sufficient elastic properties at pressures observed in the natural articular joint. For example, the polymeric insert may comprise a polyetheretherketone (PEEK), a polyetherketoneketone (PEKK), a polyetherketoneetherketoneketone (PEKEKK), or a polymethylmethacrylate (PMMA). Moreover, the polymeric insert may comprise a single polymer, a co-polymer, PrimoSpire^{™} self-reinforced polyphenylene (SRP), or a polymer blend. The polymer selection may depend upon the joint in question. For example, joints in an arm experience different overall loading levels under different motions, thus justifying a different material for the non-metallic wear layer 30 than for a knee joint. The non-metallic wear layer 30 may also or alternatively comprise another non-metallic, synthetic material that has material properties similar to a healthy meniscus or one that absorbs or distributes stresses, particularly when loaded with compressive stress or shear stress, and that resists degradation due to continuous frictional contact with the hard wear layer 50.

In another exemplary embodiment of the invention, depicted in FIG. 2A, the non-metallic wear layer 30' includes a ceramic coating 30b at the non-metallic articular surface 32, which is applied on a non-metallic substrate 30a, for example, a polymer substrate. The pressures exerted on the bone 70 are passed to the polymeric implant component 40, the hard wear layer 50, the ceramic coating 30b, the non-metallic substrate 30a, and the rigid implant component 20. The ceramic coating 30b is therefore adapted to articulate against the hard wear layer 50. However, as previously discussed, the system 10 absorbs and distributes loads. By way of example and not limitation, the ceramic coating 30b may be formed on the non-metallic substrate 30a by methods known by those skilled in the art, for example, by plasma spray, flame spray, HVOF spray, cold spray, or other spray coating technique that provides bonding without substantial degradation to the non-metallic substrate 30a. In addition, the ceramic coating 30b may also be formed by ion implantation, ion beam assisted deposition, CVD, or PVD as is known in the art.

In another exemplary embodiment, shown in FIGS. 1C and 3, the system 10 is a prosthetic implant used in total knee arthroscopy, as is known in the art for replacing the knee joint due to osteoarthritis, rheumatoid arthritis, traumatic arthritis, or other condition causing pain or limiting the functionality of the knee. While FIGS. 1C and 3 depict a prosthetic used in total knee arthroscopy, the system 10 is not limited solely to knee replacement. Other joints in humans, as well as is quadrupeds, may be replaced with the system 10. For example, in a total hip replacement, the system 10 may replace a deteriorated femoral head and acetabulum, or the system 10 may replace other joints such as those in a digit or a shoulder, or a stifle in a quadraped.

As shown in FIGS. 1C and 3, in a total knee replacement, the rigid implant component 20 may be attached to the femur and thus referred to as a femoral implant. Consequently, the polymeric implant component 40 may be attached to the tibia and thus referred to as a tibial implant. The system 10 replaces the knee joint and may substantially mimic the motion of the natural knee. More specifically, as shown in FIG. 3, the non-metallic articular surface 32 and the hard articular surface 52 are in frictional contact during femoral rollback, such as while walking, running, and jumping. While FIG. 3 depicts the rigid implant component 20 as having the non-metallic articular surface 32 divided into a medial condylar surface and a lateral condylar surface with the hard articular surface 52 similarly divided, the system 10 is not limited to this physiological configuration. For example, the non-metallic articular surface 32 may be a single surface in frictional contact with the hard articular surface 52 configured as a single surface. With respect to a total knee replacement, by way of example only, the femoral implant may comprise a metal, for example, titanium, a cobalt chrome alloy, or stainless steel. In addition, by way of example, the tibial implant may comprise polyethylene or polyetheretherketone (PEEK), polyetherketoneketone, polypropylene, or PrimoSpire^{™} self-reinforced polyphenylene (SRP).

In further respect to a total knee replacement, in one embodiment of the present invention, the hard wear layer 50, as shown in FIGS. 1 and 3 is a ceramic insert. Therefore, in FIG. 1C, the working surface 22 articulates against the ceramic insert, and, in FIG. 3, the non-metallic wear layer 30 articulates against the ceramic insert. The system 10 bears and transmits pressures from longitudinal and lateral loading by absorbing and distributing those loads through the polymeric implant component 40, the ceramic insert, the non-metallic wear layer 30, shown in FIG. 3, and the rigid implant component 20. Loads which cause motion of the femur with respect to the tibia may also cause the hard articular surface 52 to articulate against the non-metallic articular surface 32. In summary, little or no wear is observed on either the ceramic insert or non-metallic wear layer 30.

While the present invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention is therefore not limited to the specific details, representative embodiments and illustrative examples shown and described. Accordingly, departures may be made from such details without departing from the scope of the general inventive concept.

## Claims

1. An implant articular surface wear reduction system (10) comprising :
a rigid implant component (20) having a working surface (22);
a polymeric implant component (40) having a mounting surface(42); and
a hard wear layer (50) having a hard articular surface (52), whereby the hard wear layer (50) is attached to the mounting surface (42), so that the hard articular surface (52) is adapted to articulate against the working surface (22) when the rigid implant component (20) pivots relative to the polymeric implant component (40), **characterised in that** the mounting surface (42) further includes a first recess (44), and the hard wear layer (50) is positioned in the first recess (44).

2. The system (10) of claim 1 **characterised in that** a volume of polymeric material comprising the polymeric implant component (40) is greater than a volume of hard wear material comprising the hard wear layer (50).

3. The system (10) of claim 1 **characterised in that** the polymeric implant component (40) comprises a polyetheretherketone.

4. The system (10) of claim 1 **characterised in that** the rigid implant component (20) comprises a ceramic.

5. The system (10) of claim 1 **characterised in that** the polymeric implant component (40) is a tibial implant and the rigid implant component (20) is a femoral implant.

6. The system (10) of claim 1 **characterised in that** the hard wear layer (50) comprises a ceramic.

7. The system (10) of claim 1 **characterised in that** the hard articular surface (52) has a means for retaining a lubricating fluid on the hard wear layer (50).

8. The system (10) of claim 1 **characterised in that** the rigid implant component (20) is a metal.

9. The system (10) of claim 8 **characterised in that** it further includes a ceramic coating positioned on the working surface (42), whereby the hard wear layer (50) is adapted to articulate against the ceramic coating when the rigid implant component (20) pivots against the polymeric implant component (40).

10. The system (10) of claim 8 **characterised in that** a non-metallic wear layer (30) having a non-metallic articular surface (32) is attached to the working surface (22).

11. The system (10) of claim 10 **characterised in that** the working surface (22) further includes a second recess (24), and the non-metallic wear layer (30) is positioned in the second recess (24).

12. The system (10) of claim 10 **characterised in that** a volume of metallic material comprising the rigid implant component (20) is greater than a volume of non-metallic material comprising the non-metallic wear layer (30).

13. The system (10) of claim 10 **characterised in that** the non-metallic wear layer (30) comprises a ceramic.

14. The system (10) of claim 10 **characterised in that** the non-metallic wear layer (30) comprises a polymer selected from a polyetheretherketone, a polyetherketoneketone, a self-reinforced polyphenylene, a polyetherketoneetherketoneketone, or a polymethylmethacrylate, or a combination thereof.

15. The system (10) of claim 10 **characterised in that** it further includes a ceramic coating (30b) positioned on the non-metallic articular surface (32) of the non-metallic wear layer(30), whereby the hard wear layer (50) is adapted to articulate against the ceramic coating (30b) when the rigid implant component (20) pivots against the polymeric implant component (40).

16. The system (10) of claim 10 **characterised in that** the hard wear layer (50) comprises a metal.

17. The system (10) of claim 8 **characterised in that** the rigid implant component (20) comprises a titanium alloy.

18. The system (10) of claim 8 **characterised in that** the rigid implant component (20) comprises a cobalt-chromium alloy.

19. The system (10) of claim 8 **characterised in that** the rigid implant component (20) comprises a zirconium alloy.

20. The system (10) of claim 8 **characterised in that** the working surface (22) has a means for retaining a lubricating fluid on the rigid implant component (20).

## Patentansprüche

1. System (10) zur Verschleißminderung von Implantatgelenkoberflächen, das aufweist:
eine steife Implantatkomponente (20) mit einer Arbeitsfläche (22);
eine Polymerimplantatkomponente (40) mit einer Befestigungsfläche (42); und
eine harte Verschleißschicht (50) mit einer harten Gelenkoberfläche (52), wobei die harte Verschleißschicht (50) an der Befestigungsfläche (42) befestigt ist, so dass die harte Gelenkoberfläche (52) eingerichtet ist, sich gelenkig an der Arbeitsfläche (22) zu bewegen, wenn die steife Implantatkomponente (20) relativ zur Polymerimplantatkomponente (40) schwenkt, **dadurch gekennzeichnet, dass** die Befestigungsfläche (42) ferner eine erste Aussparung (44) aufweist und die harte Verschleißschicht (50) in der ersten Aussparung (44) angeordnet ist.

2. System (10) nach Anspruch 1, **dadurch gekennzeichnet, dass** ein Volumen des Polymermaterials, das die Polymerimplantatkomponente (40) aufweist, größer als ein Volumen des harten Verschleißmaterials ist, das die harte Verschleißschicht (50) aufweist.

3. System (10) nach Anspruch 1, **dadurch gekennzeichnet, dass** die Polymerimplantatkomponente (40) Polyetheretherketon aufweist.

4. System (10) nach Anspruch 1, **dadurch gekennzeichnet, dass** die steife Implantatkomponente (20) Keramik aufweist.

5. System (10) nach Anspruch 1, **dadurch gekennzeichnet, dass** die Polymerimplantatkomponente (40) ein tibiales Implantat ist und die steife Implantatkomponente (20) ein femorales Implantat ist.

6. System (10) nach Anspruch 1, **dadurch gekennzeichnet, dass** die harte Verschleißschicht (50) Keramik aufweist.

7. System (10) nach Anspruch 1, **dadurch gekennzeichnet, dass** die harte Gelenkoberfläche (52) eine Einrichtung zum Halten einer Schmierflüssigkeit auf der harten Verschleißschicht (50) aufweist.

8. System (10) nach Anspruch 1, **dadurch gekennzeichnet, dass** die steife Implantatkomponente (20) ein Metall ist.

9. System (10) nach Anspruch 8, **dadurch gekennzeichnet, dass** es ferner einen Keramiküberzug aufweist, der auf der Arbeitsfläche (42) angeordnet ist, wodurch die harte Verschleißschicht (50) eingerichtet ist, sich gelenkig an dem Keramiküberzug zu bewegen, wenn die steife Implantatkomponente (20) bezüglich der Polymerimplantatkomponente (40) schwenkt.

10. System (10) nach Anspruch 8, **dadurch gekennzeichnet, dass** eine nichtmetallische Verschleißschicht (30) mit einer nichtmetallischen Gelenkoberfläche (32) an der Arbeitsfläche (22) befestigt ist.

11. System (10) nach Anspruch 10, **dadurch gekennzeichnet, dass** die Arbeitsfläche (22) ferner eine zweite Aussparung (24) aufweist und die nichtmetallische Verschleißschicht (30) in der zweiten Aussparung (24) angeordnet ist.

12. System (10) nach Anspruch 10, **dadurch gekennzeichnet, dass** ein Volumen des metallischen Materials, das die steife Implantatkomponente (20) aufweist, größer als ein Volumen des nichtmetallischen Materials ist, das die nichtmetallische Verschleißschicht (30) aufweist.

13. System (10) nach Anspruch 10, **dadurch gekennzeichnet, dass** die nichtmetallische Verschleißschicht (30) Keramik aufweist.

14. System (10) nach Anspruch 10, **dadurch gekennzeichnet, dass** die nichtmetallische Verschleißschicht (30) ein Polymer aufweist, das aus Polyetheretherketon, Polyetherketonketon, selbstverstärktem Polyphenylen, Polyetherketonetherketonketon oder Polymethylmethacrylat oder einer Kombination davon ausgewählt ist.

15. System (10) nach Anspruch 10, **dadurch gekennzeichnet, dass** es ferner einen Keramiküberzug (30b) aufweist, der auf der nichtmetallischen Gelenkoberfläche (32) der nichtmetallischen Verschleißschicht (30) angeordnet ist, wodurch die harte Verschleißschicht (50) eingerichtet ist, sich gelenkig am Keramiküberzug (30b) zu bewegen, wenn die steife Implantatkomponente (20) bezüglich der Polymerimplantatkomponente (40) schwenkt.

16. System (10) nach Anspruch 10, **dadurch gekennzeichnet, dass** die harte Verschleißschicht (50) ein Metall aufweist.

17. System (10) nach Anspruch 8, **dadurch gekennzeichnet, dass** die steife Implantatkomponente (20) eine Titanlegierung aufweist.

18. System (10) nach Anspruch 8, **dadurch gekennzeichnet, dass** die steife Implantatkomponente (20) eine Kobalt-Chrom-Legierung aufweist.

19. System (10) nach Anspruch 8, **dadurch gekennzeichnet, dass** die steife Implantatkomponente (20) eine Zirkonlegierung aufweist.

20. System (10) nach Anspruch 8, **dadurch gekennzeichnet, dass** die Arbeitsfläche (22) eine Einrichtung zum Halten einer Schmierflüssigkeit auf der steifen Implantatkomponente (20) aufweist.

## Revendications

1. Système (10) de réduction de l'usure d'une surface articulaire d'un implant comprenant :
un composant (20) d'implant rigide ayant une surface de travail (22) ;
un composant (40) d'implant polymère ayant une surface de montage (42) ; et
une couche d'usure dure (50) ayant une surface articulaire dure (52), moyennant quoi la couche d'usure dure (50) est fixée à la surface de montage (42), de façon à ce que la surface articulaire dure (52) soit adaptée pour s'articuler contre la surface de travail (22) lorsque le composant (20) d'implant rigide pivote par rapport au composant (40) d'implant polymère, **caractérisé en ce que** la surface de montage (42) comporte en outre un premier évidement (44), et la couche d'usure dure (50) est positionnée dans le premier évidement (44).

2. Système (10) de la revendication 1 **caractérisé en ce qu'**un volume de matériau polymère comprenant le composant (40) d'implant polymère est supérieur à un volume de matériau d'usure dur comprenant la couche d'usure dure (50).

3. Système (10) de la revendication 1 **caractérisé en ce que** le composant (40) d'implant polymère comprend du polyétheréthercétone.

4. Système (10) de la revendication 1 **caractérisé en ce que** le composant (20) d'implant rigide comprend de la céramique.

5. Système (10) de la revendication 1 **caractérisé en ce que** le composant (40) d'implant polymère est un implant tibial et le composant (20) d'implant rigide est un implant fémoral.

6. Système (10) de la revendication 1 **caractérisé en ce que** la couche d'usure dure (50) comprend de la céramique.

7. Système (10) de la revendication 1 **caractérisé en ce que** la surface articulaire dure (52) a un moyen destiné à retenir un fluide de lubrification sur la couche d'usure dure (50).

8. Système (10) de la revendication 1 **caractérisé en ce que** le composant (20) d'implant rigide est un métal.

9. Système (10) de la revendication 8 **caractérisé en ce qu'**il comporte en outre un revêtement en céramique positionné sur la surface de travail (42), moyen par lequel la couche d'usure dure (50) est adaptée pour s'articuler contre le revêtement en céramique lorsque le composant (20) d'implant rigide pivote contre le composant (40) d'implant polymère.

10. Système (10) de la revendication 8 **caractérisé en ce qu'**une couche d'usure non-métallique (30) ayant une surface articulaire non-métallique (32) est fixée à la surface de travail (22).

11. Système (10) de la revendication 10 **caractérisé en ce que** la surface de travail (22) comporte en outre un deuxième évidement (24), et la couche d'usure non-métallique (30) est positionnée dans le deuxième évidement (24).

12. Système (10) de la revendication 10 **caractérisé en ce qu'**un volume d'un matériau métallique comprenant le composant (20) d'implant rigide est supérieur à un volume d'un matériau non-métallique comprenant la couche d'usure non-métallique (30).

13. Système (10) de la revendication 10 **caractérisé en ce que** la couche d'usure non-métallique (30) comprend de la céramique.

14. Système (10) de la revendication 10 **caractérisé en ce que** la couche d'usure non-métallique (30) comprend un polymère choisi parmi du polyétheréthercétone, polyéthercétonecétone, polyphénylène auto-renforcé, polyéthercétoneéthercétonecétone, ou polyméthacrylate de méthyle, ou leur combinaison.

15. Système (10) de la revendication 10 **caractérisé en ce qu'**il comporte en outre un revêtement (30b) en céramique positionné sur la surface articulaire non-métallique (32) de la couche d'usure non-métallique (30), moyen par lequel la couche d'usure dure (50) est adaptée pour articuler contre le revêtement (30b) en céramique lorsque le composant (20) d'implant rigide pivote contre le composant (40) d'implant polymère.

16. Système (10) de la revendication 10 **caractérisé en ce que** la couche d'usure dure (50) comporte un métal.

17. Système (10) de la revendication 8 **caractérisé en ce que** le composant (20) d'implant rigide comprend un alliage de titane.

18. Système (10) de la revendication 8 **caractérisé en ce que** le composant (20) d'implant rigide comprend un alliage cobalt-chrome.

19. Système (10) de la revendication 8 **caractérisé en ce que** le composant (20) d'implant rigide comprend un alliage de zirconium.

20. Système (10) de la revendication 8 **caractérisé en ce que** la surface de travail (22) a un moyen destiné à retenir un fluide de lubrification sur le composant (20) d'implant rigide.
